# EUROPEAN PATENT SPECIFICATION

(11) **EP 3 174 902 B1**
(45) Date of publication and mention of the grant of the patent: **17.04.2019**
(21) Application number: 15744591.7
(22) Date of filing: 31.07.2015
(51) Int. Cl.: C07K 16/28, A61P 37/06

(54) **AN ANTI-CD45RC ANTIBODY FOR USE AS DRUG**
ANTI-CD45RC-ANTIKÖRPER ZUR VERWENDUNG ALS MEDIKAMENT
ANTICORPS ANTI-CD45RC DESTINÉ À ÊTRE UTILISÉ EN TANT QUE MÉDICAMENT

(30) Priority: 01.08.2014 EP 14306231
(43) Date of publication of application: 07.06.2017
(73) Proprietor: Institut National de la Santé et de la Recherche Médicale (INSERM), 75013 Paris (FR); Universite de Nantes, 44000 Nantes (FR)
(72) Inventor: GUILLONNEAU, Carole, F-44093 Nantes (FR); ANEGON, Ignacio, F-44093 Nantes (FR)
(74) Representative: Inserm Transfert
(86) International application number: PCT/EP2015/067707
(87) International publication number: WO 2016/016442

(56) References cited:
- WO-A1-98/11918
- BEDKE TANJA ET AL: "Modulation of human anti-pig T cell responses by monoclonal antibodies directed to porcine CD45 molecules.", ANNALS OF TRANSPLANTATION : QUARTERLY OF THE POLISH TRANSPLANTATION SOCIETY 2003, vol. 8, no. 3, 2003, pages 35-38, XP002733174, ISSN: 1425-9524
- G. P. SPICKETT ET AL: "MRC OX-22, a monoclonal antibody that labels a new subset of T lymphocytes and reacts with the high molecular weight form of the leukocyte-common antigen", JOURNAL OF EXPERIMENTAL MEDICINE, vol. 158, no. 3, 1 September 1983 (1983-09-01), pages 795-810, XP055155372, ISSN: 0022-1007, DOI: 10.1084/jem.158.3.795
- LAURENCE ORDONEZ ET AL: "A Higher Risk of Acute Rejection of Human Kidney Allografts Can Be Predicted from the Level of CD45RC Expressed by the Recipients' CD8 T Cells", PLOS ONE, vol. 8, no. 7, 24 July 2013 (2013-07-24), page e69791, XP055154265, DOI: 10.1371/journal.pone.0069791
- LAZAROVITS A I ET AL: "PREVENTION AND REVERSAL OF RENAL ALLOGRAFT REJECTION BY ANTIBODY AGAINST CD45RB", NATURE, NATURE PUBLISHING GROUP, UNITED KINGDOM, vol. 380, no. 6576, 25 April 1996 (1996-04-25), pages 717-720, XP002052531, ISSN: 0028-0836, DOI: 10.1038/380717A0

### SEQUENCE LISTING

<110> INSERM UNIVERSITE DE NANTES
<120> AN ANTI-CD45RC ANTIBODY FOR USE AS DRUG
<130> BIO13345 - GUILLONNEAU/AS
<150> EP 14 306 231.3
   <151> 2014-08-01
<160> 2
<170> PatentIn version 3.5
<210> 1
   <211> 144
   <212> DNA
   <213> Homo sapiens
<400> 1
   atgtcccagg agagaggagt acagccagca cctttcctac agacccagtt tccccattga        60
   caaccaccct cagccttgca caccacagct ctgctgcctt acctgcacgc acctccaaca       120
   ccaccatcac agcgaacacc tcag                                              144
<210> 2
   <211> 47
   <212> PRT
   <213> Homo sapiens
<400> 2
   Cys Pro Arg Arg Glu Glu Tyr Ser Gln His Leu Ser Tyr Arg Pro Ser
   1               5                   10                  15
   Phe Pro Ile Asp Asn His Pro Gln Pro Cys Thr Pro Gln Leu Cys Cys
               20                  25                  30
   Leu Thr Cys Thr His Leu Gln His His His His Ser Glu His Leu
           35                  40                  45

## Description

### FIELD:

The present disclosure is in the field of immunotherapy.

### BACKGROUND:

One approach in the treatment of a variety of diseases is to achieve the elimination or the inactivation of pathogenic leukocytes and the potential for induction of tolerance to inactivate pathological immune responses. The prevention of cell, tissue or organ transplant rejection and the treatment of autoimmune diseases require either the induction or the restoration of immunological tolerance respectively. Overall, the breakdown of tolerance associated with autoimmune disorders and the lack of tolerance for allografts associated with transplant rejection, are thought to be primarily T-cell mediated immune responses.

In most human diseases, the identification of cellular target for monoclonal antibodies therapy represents important challenges as such therapies are increasingly used with great success notably in transplantation (1). Such new strategies also allow a specific rather than a general immunosuppression associated with the induction of regulatory T cells (Tregs) (1, 2).The protein CD45 is a highly glycosylated transmembrane tyrosine phosphatase expressed by hematopoietic cells and described as an essential regulator of T and B cell antigen receptor signaling. The cytoplasmic domain of CD45 regulates the activity of Lck and JAKs, which are essential components of the signal transduction process of antigen recognition by T and B cells. There is several isoforms of the CD45 protein, generated by alternative splicing of exons 4 (A), exon 5 (B) and exon 6 (C) (the inclusion of exons 4 and 6 in CD45 mRNA is tightly regulated whereas the inclusion of exon 5 appears stochastic). These different extracellular domains differ in size and charge (3, 4). Individuals express different levels of CD45 isoforms (5) and while the function of the different CD45 isoforms is not clear, differential expression of these isoforms have been associated with the level of activation of T cells and allows dissociation of naive vs. memory T cells (6). Importantly, this pattern of isoforms expression is highly conserved across species emphazing its functional role and importance (7).

The CD45RA, CD45RB and CD45RC isoforms are mainly expressed by naive cells while the shortest isoform CD45RO is express by activated/memory T cells. CD45RC⁺ cells also express the A and B isoforms whereas CD45RC⁻ do not (8). CD45RO activated/memory T cells can also revert and express again A, B or C isoforms (8). In general, CD4 and CD8 T cells with high molecular forms of CD45R (ie, A, B or C) expressed mRNA for ABC (low and variable), AB, BC, B and O exons. CD4 and CD8⁺ T cells CD45RO⁺ had high levels of message for exon B with variable expression of double-exons AB and BC (8).

It has been shown in mice and primates that a population of CD4 Treg can be defined as CD45RB^{low}. Treatment of mice with anti-CD45RB antibodies can prevent and reverse kidney allograft rejection (9, 10). This antibody was also shown to prevent the onset of diabetes in a non-obese diabetic mouse model (11). Comparing two different CD45RB specific mAbs, it has been found that the antibody that was effective for tolerance induction also showed the most effective peripheral cell depletion capacity (9). In addition, the tolerogenic mAb was able to stimulate tyrosine phosphatase activity on splenic lymphocyte membranes, thereby suggesting a possible immunoregulatory capacity of this antibody. Finally, they described that the mechanisms of CD45RB mAbs were either induction of apoptotic cell death or dysregulation the signaling apparatus causing lack of effector cell function followed by activation induced cell death.

CD45RC has been described in rodents and human (12). In rodents, it has been shown that both CD4⁺ and CD8⁺ T cells CD45RC⁺ are potent Th1 effector cells capable of promoting transplant rejection and organ inflammation (13, 14), while T cells expressing undetectable or low levels of CD45RC are Th2 and regulatory T cells and inhibit allograft rejection, GVHD and cell-mediated autoimmune diseases (15-17).

It has recently been shown that treatment with CD40Ig, a fusion molecule capable of blocking CD40-CD40L interaction, provides an indefinite extension of graft survival through induction of regulatory CD8⁺CD45RC^{-/low} T cells in rats (16, 18). Only these CD8⁺CD45RC^{- /low} Treg cells are able to transfer dominant donor-specific tolerance. It has also been shown that the tolerogenic effects of CD8⁺ Treg cells from CD40Ig-treated animals are dependent of the production of IFNγ, and expression of indoleamine 2,3-dioxygenase (IDO) by graft endothelial cells and dendritic cells (DC) of the spleen (16, 19, 20). In human, an increase in CD45RC^{high} CD8 T cells has been recently correlated with a decreased graft survival (21). It has also been shown that a subset of human CD45RC⁺T cells exhibit different cytokine profiles after polyclonal stimulation and that the frequency of these cells is imbalanced in patients with vasculitis (5).

The international patent application WO 98/11918 relates to the use of antibodies to CD45R leukocyte antigens for immunomodulation based on results obtained mainly with anti-CD45RB antibodies corresponding to the results described above (9, 11) and very succinctly with anti-CD45RO antibodies. Nevertheless, said application has also suggested that an anti-CD45RC antibody could be useful in the prevention and/or treatment of transplant rejection as well as treatment of autoimmune disease in generic terms. However no prophylactic or therapeutic effect has been concretely evaluated in this document. The proposed prevention and/or treatment of such disease is thus absolutely not substantiated.

Moreover, it has also been concluded fifteen years later that anti-porcine CD45RC (IgM, MIL15) were not efficient at blocking xenogeneic immune response in a MLR assay where human PBMCs were incubated with porcine PBMCs in comparison with anti-CD45 and anti-CD45RA Abs (25). Most importantly, no isotype IgM control antibodies were used in this assay and thus the small 15% inhibition of the xenogeneic responses must be attributed to non-specific binding, demonstrating that the anti-porcine CD45RC (MIL15) antibody, in contrast to the anti-CD45 (K252.IE4) and anti-CD45RA (MIL13) antibodies, did not modulate the immune xenogeneic responses and could not be used to modulate human anti-pig T cells responses after xenotransplantation.

Accordingly, it results that until now, it has never been disclosed nor even suggested to the skilled in the art that the direct administration of an anti-CD45RC antibody, in particular an anti-CD45RC monoclonal antibody may be useful in therapy, especially for preventing allogeneic transplant rejection such as GVHD in a patient in need thereof.

### SUMMARY:

The present invention relates to an isolated anti-CD45RC monoclonal antibody for use in preventing or reducing transplant rejection in a patient in need thereof, wherein the anti-CD45RC monoclonal antibody is a CD45RC+ cell depleting antibody. In particular, the present invention is defined by the claims.

### DETAILED DESCRIPTION:

The inventors fulfill this need in providing antibodies that have the capacity to decrease aggressive effector T cells or B cells while increasing tolerogenic regulatory T cells.

The present disclosure is indeed based on the discovery that induction of transplantation tolerance is obtained with a treatment with an anti-CD45RC monoclonal antibody. Accordingly, the inventors targeted the cell populations expressing CD45RC using an anti-CD45RC monoclonal antibody (OX22 clone) administered for 10 to 20 days every 2.5 days and demonstrated the potency and advantages of such strategy for allograft survival in a rat cardiac transplantation model. They showed that short-term anti-CD45RC antibody treatment resulted in permanent allograft survival with no signs of chronic rejection and associated to inhibition of effector T cells, as well as induction of regulatory T cells of increased activity and that this tolerance could be transferred to secondary irradiated recipients. Both CD4⁺ and CD8⁺ Tregs transferred tolerance. In addition, they demonstrated that anti-donor antibody responses were completely inhibited, while immune responses against cognate antigens were preserved. Altogether, they showed for the first time the potential of a short-term anti-CD45RC treatment as a new immunosuppressive therapy in transplantation capable of inducing potent active tolerogenic cellular mechanisms.

### Definitions:

Throughout the specification, several terms are employed and are defined in the following paragraphs.

As use herein, the term "CD45" (also known as CD45R or PTPRC) refers to a transmembrane glycoprotein existing in different isoforms. These distinct isoforms of CD45 differ in their extracellular domain structures which arise from alternative splicing of 3 variable exons coding for part of the CD45 extracellular region (3). The various isoforms of CD45 have different extracellular domains, but have the same transmembrane and cytoplasmic segments having two homologous, highly conserved phosphatase domains of approximately 300 residues.

As used herein, the term "CD45RC" refers to a 200-220 kD single chain type I membrane glycoprotein well known from the skilled man in the art (3). It is an exon 6 splice variant (exon C) of the tyrosine phosphatase CD45. The CD45RC isoform is expressed on B cells, majority of CD8⁺ T cells and a subset of CD4⁺ T cells, but not on myeloid cells. CD45 and its isoforms non-covalently associate with lymphocyte phosphatase-associated phosphoprotein (LPAP) on T and B lymphocytes. CD45 has been reported to be associated with several other cell surface antigens, including CD1, CD2, CD3, and CD4. CD45 is involved in signalling lymphocytes activation.

Some monoclonal antibodies (mAbs) recognize an epitope in the portion of CD45 common to all the different isoforms (anti-CD45R), while other mAbs have a restricted specificity, dependent on which of the alternatively spliced exons (A, B or C) they recognize. For example, mAbs recognizing the product of exon A are consequently designated anti-CD45RA, those recognizing the product of exon B are consequently designated anti-CD45RB and those recognizing the product of exon C are consequently designated anti-CD45RC. Accordingly, an antibody that specifically binds to CD45RC binds to a polypeptide sequence defined by SEQ ID NO: 2 and encoded by the nucleotide sequence SEQ ID NO: 1 (exon 6, isoform C) as listed in Table A:

**Table A: Nucleotide and polypeptide sequences of Exon 6**

| **Exon 6 (SEQ ID NO: 1)** | **Product of the exon 6 (SEQ ID NO: 2)** |
|---|---|
| | |

An "isolated antibody", as used herein, is intended to refer to an antibody that is substantially free of other antibodies having different antigenic specificities (e.g., an isolated antibody that specifically binds to a CD45RC protein is substantially free of antibodies that specifically bind antigens other than CD45RC proteins). An isolated antibody that specifically binds a human CD45RC protein may, however, have cross-reactivity to other antigens, such as CD45RC proteins from other species. Moreover, an isolated antibody can be substantially free of other cellular material and/or chemicals.

As used herein, the term "antibody" or "immunoglobulin" have the same meaning, and will be used equally in the present disclosure. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

Within the context of the present disclosure, the anti-CD45RC monoclonal antibody has preferably an IgG isotype (such as IgG1, IgG2, IgG3 or IgG4).

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond. The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin. The term "Fab"' refers to an antibody fragment having a molecular weight of about 50,000 and antigen binding activity, which is obtained by cutting a disulfide bond of the hinge region of the F(ab')2. A single chain Fv ("scFv") polypeptide is a covalently linked VH:: VL heterodimer which is usually expressed from a gene fusion including VH and VL encoding genes linked by a peptide-encoding linker. "dsFv" is a VH:: VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2. The term "diabodies" refers to small antibody fragments with two antigen-binding sites, which fragments comprise a heavy-chain variable domain (VH) connected to a light- chain variable domain (VL) in the same polypeptide chain (VH-VL). By using a linker that is too short to allow pairing between the two domains on the same chain, the domains are forced to pair with the complementary domains of another chain and create two antigen-binding sites.

Antibodies directed against the CD45RC surface marker can be raised according to known methods by administering the appropriate antigen or epitope (such as the peptide of SEQ ID NO: 2) to a host animal selected, e.g., from rats, pigs, cows, horses, rabbits, goats, sheep, Camelidae (camel, dromedary, llama) and mice, among others. Various adjuvants known in the art can be used to enhance antibody production. Although antibodies can be polyclonal, monoclonal antibodies are preferred. Monoclonal antibodies can be prepared and isolated using any technique that provides for the production of antibody molecules by continuous cell lines in culture. Techniques for production and isolation include but are not limited to the hybridoma technique, the human B-cell hybridoma technique and the EBV-hybridoma technique. Alternatively, techniques described for the production of single chain antibodies (see, e.g., U.S. Pat. No. 4,946,778) can be adapted to produce single chain antibodies against the CD45RC surface marker.

Useful antibodies herein disclosed also include antibody fragments including but not limited to F(ab')2 fragments, which can be generated by pepsin digestion of an intact antibody molecule, and Fab fragments, which can be generated by reducing the disulfide bridges of the F(ab')2 fragments as well as scFv and dsFv as above-mentioned. Alternatively, Fab and/or scFv expression libraries can be constructed to allow rapid identification of fragments having the desired specificity to the CD45RC surface marker. Useful antibodies herein disclosed also include different antibody formats created from these fragments, in particular formats of chimerized or humanized, multispecific and/or multivalent antibodies. The "antibody formats" as referred herein correspond to different combinations of domains and regions such as variable domains of heavy single chain antibodies (VHH) from Camelidae (camel, dromedary, llama), specifically recognizing a type of antigen.

Examples of antibodies which bind the CD45RC antigen that are contemplated by the present disclosure include the mouse monoclonal antibodies OX-22 (anti-rat CD45RC), OX-32 (anti-rat CD45RC), MT2 (anti-human CD45RC), and RP1/12 (anti-rat CD45RC).

Monoclonal antibodies directed against CD45RC are well known from the skilled man in the art such as the antibodies commercialized by Santa Cruz Biotech. Examples of antibodies which bind the CD45RC antigen that are contemplated by the present disclosure include antibodies such as the monoclonal antibody OX-22 or a derivative thereof. Such monoclonal antibody is well known from the skilled man in the art and has been initially described in Spickett et al. 1983 and is commercialized by several companies.

In one embodiment, the anti-CD45RC monoclonal antibody is an anti-human CD45RC monoclonal antibody. Examples of antibodies which bind the human CD45RC antigen that are contemplated by the present disclosure include antibodies such as the monoclonal antibody MT2 or a derivative thereof and the monoclonal antibody RP1/12 or a derivative thereof. Such monoclonal antibodies are well known from the skilled man in the art and are commercialized by several companies.

The expression "a derivative of MT2 " refers to an anti-CD45RC antibody which specifically binds to human CD45RC and which comprises the 6 CDRs of MT2.

In one embodiment, the derivative of MT2 is an antibody which comprises the VL chain and the VH chain of MT2.

In another embodiment, the derivative of MT2 is a chimeric antibody or humanized antibody, which comprises the variable domains of MT2.

The expression "a derivative of RP1/12" refers to an anti-CD45RC antibody which specifically binds to human CD45RC and which comprises the 6 CDRs of RP1/12.

In one embodiment, the derivative of RP1/12 is an antibody which comprises the VL chain and the VH chain of RP1/12.

In another embodiment, the derivative of RP1/12 is a chimeric antibody or humanized antibody, which comprises the variable domains of RP1/12.

The terms "chimeric antibody" refer to a genetically engineered fusion of parts of an animal antibody, typically a mouse antibody, with parts of a human antibody. Generally, chimeric antibodies contain approximately 33% mouse protein and 67% human protein. Developed to reduce the Human anti-animal antibodies response elicited by animal antibodies, they combine the specificity of the animal antibody with the efficient human immune system interaction of a human antibody.

Humanized antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

The human chimeric antibody herein disclosed can be produced by obtaining nucleic sequences encoding VL and VH domains as previously described, constructing a human chimeric antibody expression vector by inserting them into an expression vector for animal cell having genes encoding human antibody CH and human antibody CL, and expressing the coding sequence by introducing the expression vector into an animal cell. As the CH domain of a human chimeric antibody, it may be any region which belongs to human immunoglobulin, but those of IgG class are suitable and any one of subclasses belonging to IgG class, such as IgG1, IgG2, IgG3 and IgG4, can also be used. Also, as the CL of a human chimeric antibody, it may be any region which belongs to Ig, and those of kappa class or lambda class can be used. Methods for producing chimeric antibodies involve conventional recombinant DNA and gene transfection techniques are well known in the art (See patent documents US5,202,238; and US5,204,244).

The humanized antibody herein disclosed may be produced by obtaining nucleic acid sequences encoding CDR domains, as previously described, constructing a humanized antibody expression vector by inserting them into an expression vector for animal cell having genes encoding (i) a heavy chain constant region identical to that of a human antibody and (ii) a light chain constant region identical to that of a human antibody, and expressing the genes by introducing the expression vector into an animal cell. The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, humanized antibody expression vector of the tandem type is preferred. Examples of tandem type humanized antibody expression vector include pKANTEX93 (WO 97/10354), pEE18 and the like.

Methods for identifying CDRs of a monoclonal antibody are well known in the art (See Antibody Engineering: Methods and Protocols, 2004).

In another embodiment, amino acid sequence modification(s) of the MT2 or RP1/12 antibody are also contemplated. For example, it may be desirable to improve the binding affinity and/or other biological properties of the antibody. It is known that when a humanized antibody is produced by simply grafting only CDRs in VH and VL of an antibody derived from a non-human animal in FRs of the VH and VL of a human antibody, the antigen binding activity is reduced in comparison with that of the original antibody derived from a non-human animal. It is considered that several amino acid residues of the VH and VL of the non- human antibody, not only in CDRs but also in FRs, are directly or indirectly associated with the antigen binding activity. Hence, substitution of these amino acid residues with different amino acid residues derived from FRs of the VH and VL of the human antibody would reduce of the binding activity. In order to resolve the problem, in antibodies grafted with human CDR, attempts have to be made to identify, among amino acid sequences of the FR of the VH and VL of human antibodies, an amino acid residue which is directly associated with binding to the antibody, or which interacts with an amino acid residue of CDR, or which maintains the three-dimensional structure of the antibody and which is directly associated with binding to the antigen. The reduced antigen binding activity could be increased by replacing the identified amino acids with amino acid residues of the original antibody derived from a non- human animal. Modifications and changes may be made in the structure of the antibodies herein disclosed, and in the DNA sequences encoding them, and still obtain a functional molecule that encodes an antibody with desirable characteristics. In making the changes in the amino sequences, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: iso leucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

In a preferred embodiment, the antibodies are fully human monoclonal antibodies. Such fully human monoclonal antibodies directed against human CD45RC can be generated using transgenic or transchromosomic mice carrying parts of the human immune system rather than the mouse system. These transgenic and transchromosomic mice include mice referred to herein as the HuMAb Mouse® and KM Mouse®, respectively, and are collectively referred to herein as "human Ig mice."

The HuMAb Mouse® (Medarex®, Inc.) contains human immunoglobulin gene miniloci that encode unrearranged human heavy (µ and γ) and κ light chain immunoglobulin sequences, together with targeted mutations that inactivate the endogenous µ and κ chain loci (see e.g., Lonberg et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGK monoclonal antibodies (Lonberg et al. (1994), supra; reviewed in Lonberg (1994) Handbook of Experimental Pharmacology 113:49-101 : Lonberg, N. and Huszar, D. (1995) Intern. Rev. Immunol. 13: 65-93, and Harding and Lonberg (1995) Ann. N Y. Acad. Sci. 764:536-546). Preparation and use of the HuMAb Mouse®, and the genomic modifications carried by such mice, is further described in Taylor et al. (1992) Nucleic Acids Research 20:6287-6295; Chen et al. (1993) International Immunology 5: 647-656; Tuaillon et al. (1993) Proc. Natl. Acad. Sci. USA 90:3720-3724; Choi et al. (1993) Nature Genetics 4: 117 -123; Chen et al. (1993) EMBO J. 12: 821-830; Tuaillon et al. (1994) J. Immunol. 152:2912-2920; Taylor et al. (1994) International Immunology 6: 579-591 ; and Fishwild et al. (1996) Nature Biotechnology 14: 845-851. See further, U.S. Patent Nos. 5,545,806; 5,569,825; 5,625, 126; 5,633,425; 5,789,650; 877,397; 5,661,016; 5,814,318; 5,874,299; 5,770,429; and 5,545,807; PCT Publication Nos. WO 92/03918; WO 93/12227; WO 94/25585; WO 97/13852; WO 98/24884; WO 99/45962 and WO 01/14424.

Fully human antibodies herein disclosed can also be raised using a mouse that carries human immunoglobulin sequences on transgenes and transchomosomes, such as a mouse that carries a human heavy chain transgene and a human light chain transchromosome. This mouse is referred to herein as a "KM mouse" and is described in detail in PCT Publication WO 02/43478. A modified form of this mouse, which further comprises a homozygous disruption of the endogenous FcyRIIB receptor gene, is also described in PCT Publication WO 02/43478 and referred to herein as a "KM/FCGR2D mouse®." In addition, mice with either the HCo7 or HCol2 heavy chain transgenes or both can be used.

Additional transgenic animal embodiments include the Xenomouse (Abgenix, Inc., U.S. Patent Nos. 5,939,598; 6,075, 181; 6,114,598; 6,150,584 and 6,162,963). Further embodiments include "TC mice" (Tomizuka et al. (2000) Proc. Natl. Acad. Set USA 97:722-727) and cows carrying human heavy and light chain transchromosomes (Kuroiwa et al. (2002) Nature Biotechnology 20:889-894; PCT Publication WO 02/092812).

Still additional transgenic animal embodiments include the OmniRat™ (OMT, Inc., PCT Publication Nos. WO2008/151081; Geurts et al. (2009) Science. Jul 24;325(5939):433 and Menoret at al. (2010) Eur J Immunol. Oct;40(10):2932-41.

In one embodiment, the anti-CD45RC antibody is an antibody modulating the immune response by competing with CD45RC for its binding site *in vivo.*

Such anti-CD45RC modulating antibody includes antibody fragments and different antibody formats created from these fragments, in particular formats of chimerized or humanized, multispecific and/or multivalent antibodies.

As herein disclosed, the anti-CD45RC antibody is a CD45RC⁺ cell depleting antibody.

As used herein, the term "CD45RC⁺ cell depleting antibodies" are defined as those antibodies which bind to a CD45RC⁺ cell surface marker on the surface of CD45RC⁺ cells (preferably CD45RC) and mediate their destruction or depletion (i.e. reduce circulating CD45RC⁺ T cell levels in a patient treated therewith) when they bind to said cell surface marker. Such depletion may be achieved via various mechanisms such as antibody-dependent cell mediated cytotoxicity (ADCC) and/or complement dependent cytotoxicity (CDC), inhibition of CD45RC⁺ cell proliferation and/or induction of CD45RC⁺ cell death (e.g. via apoptosis). The CD45RC⁺ cell depleting antibodies, more preferably an anti-CD45RC antibody, optionally conjugated with or fused to a cytotoxic agent. The term includes antibody fragments and different antibody formats created from these fragments, in particular formats of chimerized or humanized, multispecific and/or multivalent antibodies. The "antibody formats" correspond to different combinations of domains and regions such as variable domains of heavy single chain antibodies (VHH) from Camelidae (camel, dromedary, llama), specifically recognizing a type of antigen.

As used herein, the terms "CD45RC⁺ cell surface marker" or "CD45RC⁺ cell antigen" refer to an antigen expressed on the surface of a CD45RC⁺ cells (including T and B cells) which can be targeted with an anti-CD45RC agent which binds thereto (such as an antibody or an aptamer). Exemplary CD45RC⁺ T cell surface markers include but are not limited to the CD45RC as previously described or other antigens that characterize said population of T cells. The CD45RC⁺ T cells surface marker of particular interest is preferentially expressed on CD45RC⁺ T cells compared to other non-CD45RC⁺ T cells of a mammal.

Then after raising antibodies directed against the CD45RC cell surface marker as above described, the skilled man in the art can easily select those that deplete CD45RC⁺ cells, for example those that deplete CD45RC⁺ cells via antibody-dependent cell mediated cytotoxicity (ADCC), complement dependent cytotoxicity (CDC), inhibition of CD45RC⁺ cell proliferation or induction of CD45RC⁺ cell death (e.g. via apoptosis).

The term "antibody-dependent cell-mediated cytotoxicity" (ADCC) refers to a form of cytotoxicity in which secreted antibodies bound onto Fc receptors (FcRs) present on certain cytotoxic cells (e.g. NK cells, neutrophils, monocytes and macrophages) enable these cytotoxic effector cells to bind specifically to an antigen-bearing target cell and subsequently kill the target cell. To assess ADCC activity of a molecule of interest, an *in vitro* ADCC assay, such as that described in US Patent No. 5,500,362 or 5,821,337 may be performed.

The term "complement dependent cytotoxicity" (CDC) refers to the lysis of a target cell in the presence of complement. Activation of the classical complement pathway is initiated by the binding of the first component of the complement system to antibodies which are bound to their cognate antigen. To assess complement activation, a CDC assay, e.g. as described in Gazzano-Santoro et al. (1997) may be performed.

In a particular embodiment, the anti-CD45RC antibody may consist in an antibody directed against the CD45RC surface marker which is conjugated to a cytotoxic agent or a growth inhibitory agent.

Accordingly, the present disclosure contemplates the use of immunoconjugates comprising an antibody against the CD45RC surface marker conjugated to a cytotoxic agent or a growth inhibitory agent. A "growth inhibitory agent" when used herein refers to a compound or composition which inhibits growth of a cell, especially CD45RC⁺ cells, either *in vitro* or *in vivo.* Examples of growth inhibitory agents include agents that block cell cycle progression, such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, and 5-fluorouracil. The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Rel⁸⁶ , Rel⁸⁸, Sml⁵³ , Bi²¹², P³², and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, e.g., gelonin, ricin, saporin, and the various antitumor or anticancer agents disclosed below.

Conjugation of the antibodies herein disclosed with cytotoxic agents or growth inhibitory agents may be made using a variety of bifunctional protein coupling agents including but not limited to N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p- diazoniumbenzoyl)-ethylenediamine), diisocyanates (such as tolyene 2,6diisocyanate), and bis-active fluorine compounds (such as 1,5-difluoro-2,4- dinitrobenzene). For example, a ricin immunotoxin can be prepared as described in Vitetta et al (1987). Carbon labeled 1- isothiocyanatobenzyl methyldiethylene triaminepentaacetic acid (MX-DTPA) is an exemplary chelating agent for conjugation of radio nucleotide to the antibody (WO 94/11026).

### Therapeutic methods and uses:

Herein disclosed are methods and compositions (such as pharmaceutical compositions) for use in inducing immune tolerance in a patient in need thereof.

Herein disclosed are methods and compositions for use in preventing or reducing transplant rejection in a patient in need thereof.

Herein disclosed are methods and compositions for use in preventing or treating autoimmune diseases, unwanted immune response against therapeutic proteins, allergies and lymphoma or cancer, which are associated with CD45RC⁺ cells in a patient thereof.

Herein disclosed is an isolated anti-CD45RC antibody for use as drug.

In a particular embodiment, the anti-CD45RC antibody is an anti-CD45RC monoclonal antibody, more particularly an anti-human CD45RC monoclonal antibody as described above.

Herein disclosed is an isolated anti-CD45RC antibody for use in inducing immune tolerance in a patient in need thereof.

In a particular embodiment, the anti-CD45RC antibody is an anti-CD45RC monoclonal antibody, more particularly an anti-human CD45RC monoclonal antibody as described above.

As used herein, the term "immune tolerance" refers to a state of unresponsiveness of the immune system to specific substances or tissues that have the capacity to elicit an immune response while preserving immune responses against other substances or tissues.

As used herein, the term "immune response" includes T cell mediated and/or B cell mediated immune responses. Exemplary immune responses include T cell responses, e.g., cytokine production and cellular cytotoxicity, in addition, the term immune response includes immune responses that are indirectly effected by T cell activation, e.g., antibody production (humoral responses) and activation of cytokine responsive cells, e.g., macrophages. Immune cells involved in the immune response include lymphocytes, such as B cells and T cells (CD4⁺, CD8⁺, Th1 and Th2 cells); antigen presenting cells (e.g. professional antigen presenting cells such as dendritic cells); natural killer cells; myeloid cells, such as macrophages, eosinophils, mast cells, basophils, and granulocytes.

Herein disclosed is an isolated anti-CD45RC antibody for use in expanding and/or potentiating regulatory T cells in a patient in need thereof.

As used herein, the term "regulatory T cells" (Tregs) refers to T cells that suppress an abnormal or excessive immune response and play a role in immune tolerance. The regulatory T cells are typically forkhead box P3 (Foxp3⁺) regulatory T cells" and "CD45RC^{low} cells". As used herein, the terms "forkhead box P3 (Foxp3⁺) regulatory T cells" or "Foxp3⁺ Treg cells" refer to 2-10% of CD4⁺ and CD8⁺ T cells in humans and rodents (rats or mice) whose the characteristic marker is the transcription factor Foxp3.

As used herein, the term "expanding" refers to the process of converting and/or amplifying a given population of cells (e.g. immune cells such as Treg cells).

As used herein, the term "potentiating" refers to the process of increasing the function of a given population of cells (e.g. increasing the suppressive capacity of Treg cells as detailed in the Section Examples below).

In one embodiment, said Treg cells are Foxp3⁺ and/or CD45RC^{low} Treg cells.

Herein disclosed is an isolated anti-CD45RC antibody for use in preventing or reducing transplant rejection in a patient in need thereof.

In a particular embodiment, the anti-CD45RC antibody is an anti-CD45RC monoclonal antibody, more particularly an anti-human CD45RC monoclonal antibody as described above.

As used herein, the term "preventing or reducing transplant rejection" is meant to encompass prevention or inhibition of immune transplant rejection, as well as delaying the onset or the progression of immune transplant rejection. The term is also meant to encompass prolonging survival of a transplant in a patient, or reversing failure of a transplant in a patient. Further, the term is meant to encompass ameliorating a symptom of an immune transplant rejection, including, for example, ameliorating an immunological complication associated with immune rejection, such as for example, interstitial fibrosis, chronic graft arteriosclerosis, or vasculitis.

As used herein, the term "transplant rejection" encompasses both acute and chronic transplant rejection. "Acute rejection" is the rejection by the immune system of a tissue transplant recipient when the transplanted tissue is immunologically foreign. Acute rejection is characterized by infiltration of the transplant tissue by immune cells of the recipient, which carry out their effector function and destroy the transplant tissue. The onset of acute rejection is rapid and generally occurs in humans within a few weeks after transplant surgery. Generally, acute rejection can be inhibited or suppressed with immunosuppressive drugs such as rapamycin, cyclosporin, anti-CD40L monoclonal antibody and the like. "Chronic rejection" generally occurs in humans within several months to years after engraftment, even in the presence of successful immunosuppression of acute rejection. Fibrosis is a common factor in chronic rejection of all types of organ transplants.

The term "transplantation" and variations thereof refers to the insertion of a transplant (also called graft) into a recipient, whether the transplantation is syngeneic (where the donor and recipient are genetically identical), allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, including animals from phylogenically widely separated species, for example, a baboon heart being transplanted into a human host.

In a particular embodiment, the transplant rejection is an allogeneic transplant rejection. Accordingly, in one embodiment, the donor of the transplant is a human. The donor of the transplant can be a living donor or a deceased donor, namely a cadaveric donor.

In one embodiment, the transplant is an organ, a tissue or cells.

As used herein, the term "organ" refers to a solid vascularized organ that performs a specific function or group of functions within an organism. The term organ includes, but is not limited to, heart, lung, kidney, liver, pancreas, skin, uterus, bone, cartilage, small or large bowel, bladder, brain, breast, blood vessels, esophagus, fallopian tube, gallbladder, ovaries, pancreas, prostate, placenta, spinal cord, limb including upper and lower, spleen, stomach, testes, thymus, thyroid, trachea, ureter, urethra, uterus. As used herein, the term "tissue" refers to any type of tissue in human or animals, and includes, but is not limited to, vascular tissue, skin tissue, hepatic tissue, pancreatic tissue, neural tissue, urogenital tissue, gastrointestinal tissue, skeletal tissue including bone and cartilage, adipose tissue, connective tissue including tendons and ligaments, amniotic tissue, chorionic tissue, dura, pericardia, muscle tissue, glandular tissue, facial tissue, ophthalmic tissue.

In a particular embodiment, the transplant is a cardiac allotransplant.

As used herein, the term "cells" refers to a composition enriched for cells of interest, preferably a composition comprising at least 30%, preferably at least 50%, even more preferably at least 65% of said cells.

In certain embodiments the cells are selected from the group consisting of multipotent hematopoietic stem cells derived from bone marrow, peripheral blood, or umbilical cord blood; or pluripotent (i.e. embryonic stem cells (ES) or induced pluripotent stem cells (iPS)) or multipotent stem cell-derived differentiated cells of different cell lineages such as cardiomyocytes, beta-pancreatic cells, hepatocytes, neurons, etc...

In one embodiment, the cell composition is used for allogeneic hematopoietic stem cell transplantation (HSCT) and thus comprises multipotent hematopoietic stem cells, usually derived from bone marrow, peripheral blood, or umbilical cord blood.

HSCT can be curative for patients with leukemia and lymphomas. However, an important limitation of allogeneic HCT is the development of graft versus host disease (GVHD), which occurs in a severe form in about 30-50% of humans who receive this therapy.

Antibodies herein disclosed are useful in preventing or reducing Graft-versus-Host-Disease (GvHD).

Accordingly, in one embodiment, the patient in need thereof is affected with a disease selected from the group consisting of acute myeloid leukemia (AML); acute lymphoid leukemia (ALL); chronic myeloid leukemia (CML); myelodysplasia syndrome (MDS) / myeloproliferative syndrome; lymphomas such as Hodgkin and non-Hodgkin lymphomas, chronic lymphatic leukemia (CLL) and multiple myeloma.

In another aspect, herein disclosed is an isolated anti-CD45RC antibody for use in preventing or treating autoimmune diseases, unwanted immune responses against proteins expressed in the course of gene therapy or therapeutic proteins, allergies and lymphoma or cancer which are associated with CD45RC⁺ cells in a patient thereof.

In a particular embodiment, the anti-CD45RC antibody is an anti-CD45RC monoclonal antibody as described above.

As used herein, the terms "prevent", "preventing" and "prevention" refer to the administration of therapy to an individual who may ultimately manifest at least one symptom of a disease, disorder, or condition, but who has not yet done so, to reduce the chance that the individual will develop the symptom of the disease, disorder, or condition over a given period of time. Such a reduction may be reflected, for example, in a delayed onset of the at least one symptom of the disease, disorder, or condition in the patient.

As used herein, the terms "treat", "treating" or "treatment" refers to the administration of therapy to an individual in an attempt to reduce the frequency and/or severity of symptoms of a disease, defect, disorder, or adverse condition of a patient.

As used herein, the term "autoimmune disease" refers to a disease in which the immune system produces an immune response (for example, a B-cell or a T-cell response) against an antigen that is part of the normal host (that is an auto-antigen), with consequent injury to tissues. In an autoimmune disease, the immune system of the host fails to recognize a particular antigen as "self" and an immune reaction is mounted against the host's tissues expressing the antigen.

Exemplary autoimmune diseases affecting humans include rheumatoid arthritis, juvenile oligoarthritis, collagen-induced arthritis, adjuvant-induced arthritis, Sjogren's syndrome, multiple sclerosis, experimental autoimmune encephalomyelitis, inflammatory bowel disease (for example, Crohn's disease and ulcerative colitis), autoimmune gastric atrophy, pemphigus vulgaris, psoriasis, vitiligo, type 1 diabetes, non-obese diabetes, myasthenia gravis, Grave's disease, Hashimoto's thyroiditis, sclerosing cholangitis, sclerosing sialadenitis, systemic lupus erythematosis, autoimmune thrombocytopenia purpura, Goodpasture's syndrome, Addison's disease, systemic sclerosis, polymyositis, dermatomyositis, acquired hemophilia, thrombotic thrombocytopenic purpura and the like.

As used herein, the term "unwanted immune response against a therapeutic protein " refers to any unwanted immune reaction directed to proteins expressed in the course of gene therapy, and/or therapeutic proteins, such as factor VIII (hemophilia A) and other coagulation factors, enzyme replacement therapies, monoclonal antibodies (e.g. natalizumab, rituximab, infliximab), polyclonal antibodies, enzymes or cytokines (e.g. IFN *β* ).

For instance, this approach can indeed be applied to suppress an immune response, especially to prevent immune reactions to specific proteins when their expression is restored by gene therapy in individuals with corresponding genetic deficiencies. Thus, an isolated anti-CD45RC antibody herein disclosed may be used to prevent immune reactivity towards proteins normally absent in the patient due to mutations, while their reconstitution is achieved by gene therapy.

Moreover, protein therapy is an area of medical innovation that is becoming more widespread, and involves the application of proteins, such as enzymes, antibodies or cytokines, directly to patients as therapeutic products. One of the major hurdles in delivery of such medicaments involves the immune responses directed against the therapeutic protein themselves. Administration of protein-based therapeutics is often accompanied by administration of immune suppressants, which are used in order to facilitate a longer lifetime of the protein and therefore increased uptake of the protein into the cells and tissues of the organism. General immune suppressants can however be disadvantageous due to the unspecific nature of the immune suppression that is carried out, resulting in unwanted side effects in the patient. Therefore, this approach can be applied to suppress an immune response against therapeutic proteins and peptides, such as therapeutic antibodies, cytokines, enzymes or any other protein administered to a patient.

As used herein, the term "allergy" or "allergies" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees.

### Pharmaceutical compositions

Herein disclosed is also a pharmaceutical composition comprising an isolated anti-CD45RC antibody herein disclosed such as an anti-CD45RC monoclonal antibody.

In one embodiment, the pharmaceutical composition comprises an isolated anti-human CD45RC monoclonal antibody.

In one particular embodiment, the pharmaceutical composition comprises an isolated anti-CD45RC monoclonal antibody selected from the group consisting of MT2 or a derivative thereof, RP1/12 or a derivative thereof and a pharmaceutically acceptable excipient.

Therefore, an antibody herein disclosed may be combined with pharmaceutically acceptable excipients, and optionally sustained-release matrices, such as biodegradable polymers, to form therapeutic compositions. "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semisolid or liquid filler, diluent, encapsulating material or formulation auxiliary of any type. The form of the pharmaceutical compositions, the route of administration, the dosage and the regimen naturally depend upon the condition to be treated, the severity of the illness, the age, weight, and sex of the patient, etc. The pharmaceutical compositions herein disclosed can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The doses used for the administration can be adapted as a function of various parameters, and in particular as a function of the mode of administration used, of the relevant pathology, or alternatively of the desired duration of treatment. To prepare pharmaceutical compositions, an effective amount of the antibody may be dissolved or dispersed in a pharmaceutically acceptable carrier or aqueous medium. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Dosages to be administered depend on individual needs, on the desired effect and the chosen route of administration. It is understood that the dosage administered will be dependent upon the age, sex, health, and weight of the recipient, concurrent treatment, if any, frequency of treatment, and the nature of the effect desired. The total dose required for each treatment may be administered by multiple doses or in a single dose.

The doses used for the administration can be adapted as a function of various parameters, and in particular as a function of the mode of administration used, of the relevant pathology, or alternatively of the desired duration of treatment. For example, it is well within the skill of the art to start doses of the antibodies at levels lower than those required to achieve the desired therapeutic effect and to gradually increase the dosage until the desired effect is achieved. However, the daily dosage of the antibodies may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 10 mg/kg of body weight per day.

The pharmaceutical composition herein disclosed may further comprise an immunosuppressive drug.

In one embodiment, the immunosuppressive drug is selected from the group consisting of cytostatics such as mammalian target of rapamycin (mTOR) inhibitors and rapamycin (sirolimus); alkylating agents (cyclophosphamide) and antimetabolites (azathioprine, mercaptopurine and methotrexate); therapeutic antibodies (such as anti-CD40L monoclonal antibodies, anti-IL-2R monoclonal antibodies, anti-CD3 monoclonal antibodies, antilymphocyte globulin (ALG) and anti-thymocyte globulin (ATG)); calcineurin inhibitors (cyclosporine); glucocorticoids and mycophenolate mofetil.

Herein disclosed are also methods of *in vivo* induction of tolerance. The methods include pre-treatment *in vivo* therapies to prevent rejection of transplanted organs, tissues or cells and post-transplant *in vivo* therapies to reverse an immune response.

Herein disclosed is a method for inducing immune tolerance in a patient in need thereof, comprising a step of administering to said patient a therapeutically effective amount of an anti-CD45RC antibody (such as an anti-CD45RC monoclonal antibody).

Herein disclosed is a method for expanding and/or potentiating regulatory T cells in a patient in need thereof, comprising a step of administering to said patient a therapeutically effective amount of an anti-CD45RC antibody (such as an anti-CD45RC monoclonal antibody).

Herein disclosed is a method for preventing or reducing transplant rejection in a patient in need thereof, comprising a step of administering to said patient a therapeutically effective amount of an anti-CD45RC antibody (such as an anti-CD45RC monoclonal antibody) previously and/or simultaneously and/or subsequently to the transplantation.

In a particular embodiment, the transplant rejection is an allogeneic transplant rejection.

Herein disclosed is a method for preventing or reducing GVHD in a patient in need thereof, comprising a step of administering to said patient a therapeutically effective amount of an anti-CD45RC antibody (such as an anti-CD45RC monoclonal antibody) previously and/or simultaneously and/or subsequently to the allogeneic hematopoietic stem cell transplantation (HSCT).

As used herein, the term "previously" means that the antibody of interest is administered to the recipient patient before the transplantation, for instance 20, 15, 10, 5 days before the transplantation.

As used herein, the term "simultaneously" means that the antibody of interest is administered to the recipient patient the same day that the transplantation.

As used herein, the term "subsequently" means that the antibody of interest is administered to the recipient patient after the transplantation, for instance 2, 3, 4, 5, 6 or 7 days following the transplantation.

Herein disclosed is a method for improving survival of the transplant in a transplanted patient (recipient), said method comprising a step of administering to said patient a therapeutically effective amount of an anti-CD45RC antibody (such as an anti-CD45RC monoclonal antibody).

Herein disclosed is a method for preventing or reducing GVHD in a patient in need thereof, comprising the steps of (a) pre-treating the allogeneic hematopoietic stem cells (or a hematopoietic stem cell graft) with a therapeutically effective amount of an anti-CD45RC antibody; and (b) administering to the patient in need thereof the pre-treated allogeneic hematopoietic stem cells obtained at step (a).

Accordingly, herein disclosed is a population of allogeneic hematopoietic stem cells (or a hematopoietic stem cell graft) treated with an anti-CD45RC antibody.

Herein disclosed is a method for preventing or treating autoimmune diseases, unwanted immune response against therapeutic proteins, allergies and lymphoma or cancer which are associated with CD45RC⁺ cells in a patient in need thereof, comprising a step of administering to said patient a therapeutically effective amount of an anti-CD45RC antibody (such as an anti-CD45RC monoclonal antibody).

By "therapeutically effective amount" is meant an amount sufficient to achieve a concentration of antibodies which is capable of preventing, treating or slowing down the disease to be treated. Such concentrations can be routinely determined by those of skilled in the art. The amount of the polypeptide actually administered will typically be determined by a physician or a veterinarian, in the light of the relevant circumstances, including the condition to be treated, the chosen route of administration, the actual compound administered, the age, weight, and response of the patient, the severity of the subject's symptoms, and the like. It will also be appreciated by those of skilled in the art that the dosage may be dependent on the stability of the administered polypeptide.

In one embodiment, the treatment with an anti-CD45RC antibody (such as an anti-CD45RC monoclonal antibody) is administered in more than one cycle, i.e. the administration of an anti-CD45RC antibody (such as an anti-CD45RC monoclonal antibody) is repeated at least once.

For example, 2 to 10 cycles or even more, depending on the specific patient status and response, may be administered. The intervals, i.e. the time between the start of two subsequent cycles, are typically several days.

### Kit-of-part compositions:

The anti-CD45RC antibody (such as an anti-CD45RC monoclonal antibody) and the immunusuppressive drug may be combined within one formulation and administered simultaneously. However, they may also be administered separately, using separate compositions. It is further noted that they may be administered at different times.

Herein disclosed is a kit-of-part composition comprising an isolated anti-CD45RC antibody and an immunosuppressive drug.

Herein disclosed is a kit-of-part composition comprising an anti-CD45RC antibody and an immunosuppressive drug for use in preventing or reducing transplant rejection in a patient in need thereof.

In a particular embodiment, the transplant rejection is an allogeneic transplant rejection.

Herein disclosed is a kit-of-part composition comprising an anti-CD45RC antibody and an immunosuppressive drug for use in preventing or treating autoimmune diseases, alloimmune responses, allergies and lymphoma or cancer which are associated with CD45RC⁺ cells in a patient in need thereof.

The terms "kit", "product" or "combined preparation", as used herein, define especially a "kit-of-parts" in the sense that the combination partners as defined above can be dosed independently or by use of different fixed combinations with distinguished amounts of the combination partners, i.e. simultaneously or at different time points. The parts of the kit of parts can then, e.g., be administered simultaneously or chronologically staggered, that is at different time points and with equal or different time intervals for any part of the kit of parts. The ratio of the total amounts of the combination partners to be administered in the combined preparation can be varied. The combination partners can be administered by the same route or by different routes. When the administration is sequential, the first partner may be for instance administered 1, 2, 3, 4, 5, 6, 7, days before the second partner.

The present disclosure will be further illustrated by the following figures and examples. However, these examples and figures should not be interpreted in any way as limiting the scope of the present disclosure.

### FIGURES:

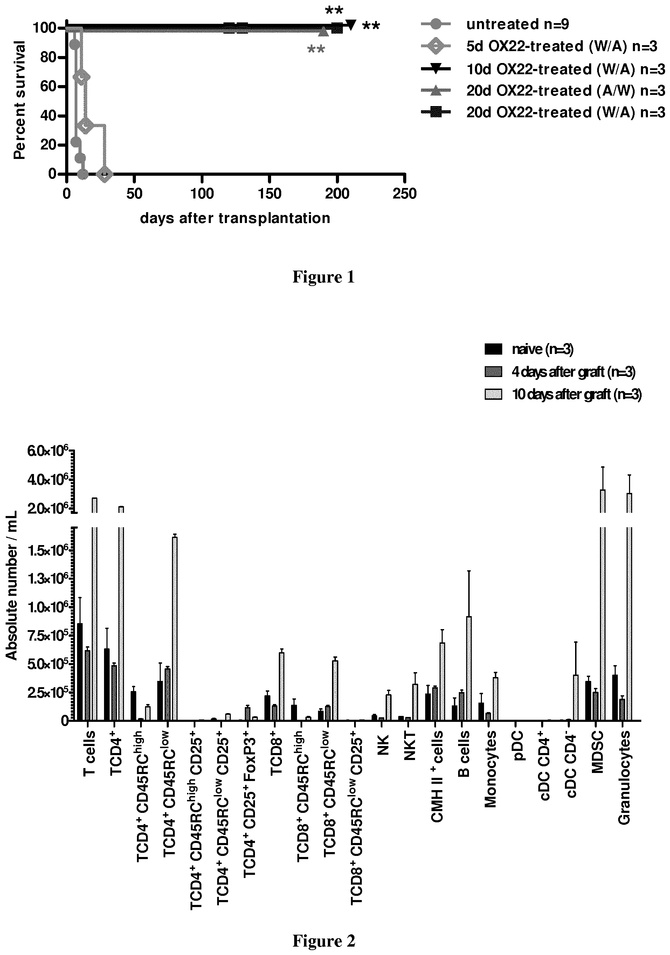

**Figure 1****: Tolerance induction after anti-CD45RC short-term treatment.** Recipients were either untreated (n=9), treated 5 days (n=3), 10 days (n=3) or 20 days (n=3) in the LEW.1W/LEW.1A strain combination or untreated (n=4) or treated 20 days in the LEW.1A/LEW.1W strain combination. Anti-CD45RC mAbs (OX22) was given i.v at 0.8 mg/kg/injection. **p<0.01 versus untreated.
**Figure 2****: Lymphocyte phenotyping in the blood during and following 10 days of anti-CD45RC treatment.** PBMCs were recovered at day 0, 4, and 10 from anti-CD45RC-treated recipients and analyzed by flow cytometry for absolute number of sub-populations.

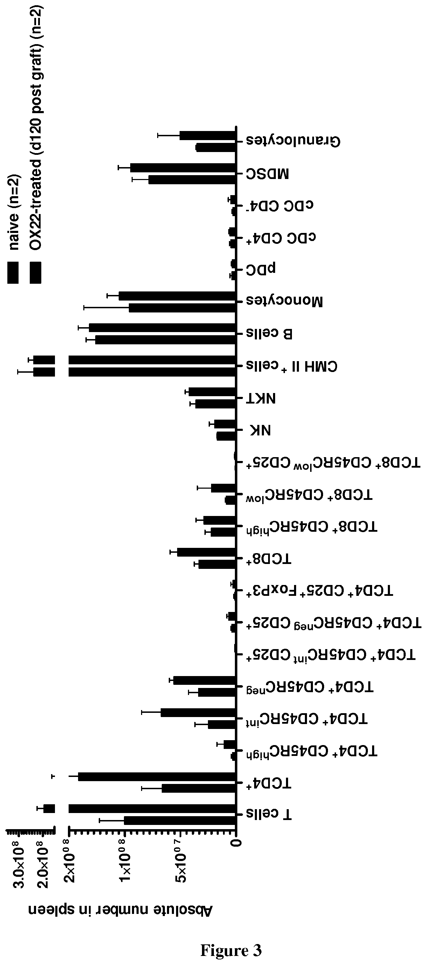

**Figure 3****: Regulatory cells are increased in the spleen of long-surviving anti-CD45RC-treated recipients.** Splenocytes were recovered at day 120 from anti-CD45RC-treated recipients compared to naive splenocytes and analyzed by flow cytometry for absolute numbers of sub-populations.

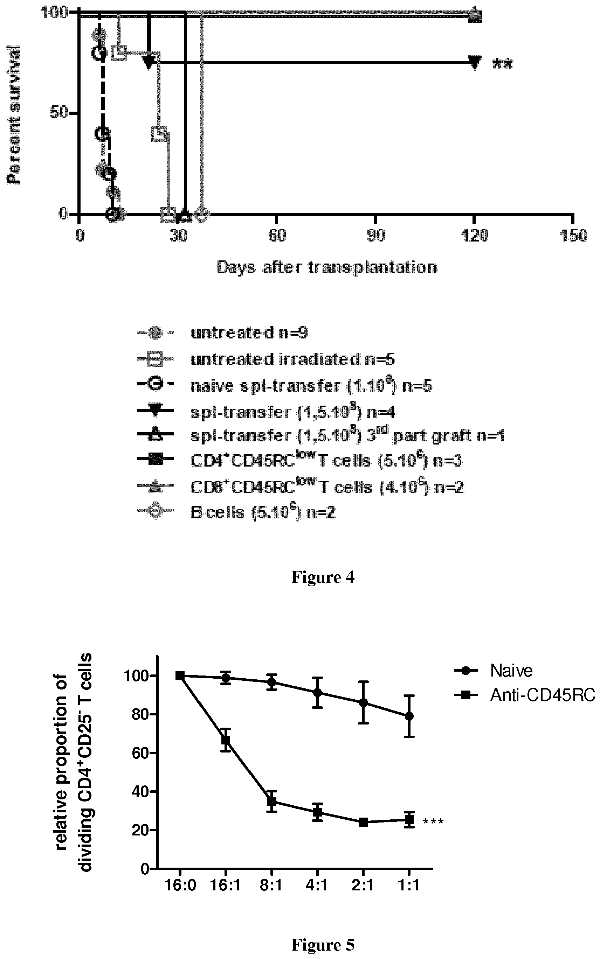

**Figure 4****: Adoptive transfer of tolerance.** LEW.1A recipients were sublethally irradiated (4.5 Gy) at day -1 and received heart allografts and i.v. injections at day 0 of splenocytes or sorted-subpopulations from long-surviving anti-CD45RC treated recipients. Graft survival was monitored by abdominal palpation.
**Figure 5****: Increased suppressive capacity of CD8⁺CD45RC^{-/low} Tregs following anti-CD45RC-treatment.** CFSE-labeled LEW.1A dividing CD4⁺CD25⁻ T cells stimulated with donor LEW.1W pDCs were analyzed after 6 days of culture in the absence or presence of d120 LEW.1A naive or anti-CD45RC-treated CD8⁺CD45RC^{-/low} Tregs at different effector/suppressor ratio.

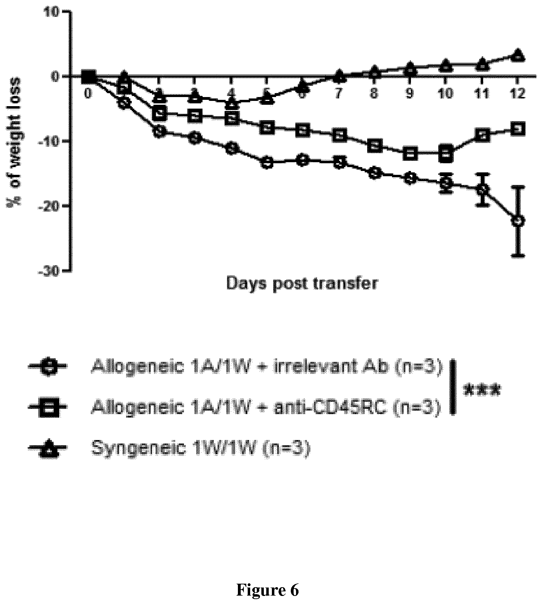

**Figure 6****: Anti-CD45RC mAb treatment reduces lethality and weight loss in a model of GVHD.** Rats receiving 200.10⁶ splenocytes were treated with anti-CD45RC mAb or isotype control to assess potency for preventing GVHD. Average weight loss (percentage of initial) for rats surviving on a given day was shown +/-SEM. ***, p< 0.001. n = 3.

### EXAMPLE 1: Short-term anti-CD45RC antibody treatment results in transplantation tolerance associated with induction of potent regulatory cells.

### Material & Methods

**Animals and cardiac transplantation models:** Heart allotransplantation were performed between whole MHC incompatible male LEW-1W and LEW-1A rats as previously described (16, 22, 23). Heart survival was evaluated by palpation through the abdominal wall and heart beating was graded from +++ to.

The experiments were approved by the regional ethical committee for animal experimentation.

**Anti-CD45RC administration:** The OX22 (IgG1 anti-rat CD45RC) mouse hybridoma was used to produce the anti-CD45RC monoclonal antibody. Anti-CD45RC mAbs were given at a dose of 0.8 mg/kg/2.5days i.v. from day 0 to 5, 10 or 20 post-transplantation. Control IgG1 (3G8, anti-human CD16, with no cross-reaction with the rat) was used at the same dose.

**Histopathology studies:** Tissue specimens were analyzed for chronic rejection at day 120 post-transplantation as previously described. Briefly, tissues were fixed in paraformaldhéhyde, paraffin embedded, cut in 5µm-thick sections and stained with hematoxylin-eosin-saffron.(24)

**Lymphocyte preparation, isolation of subpopulations and adoptive transfert:** Spleen were harvested at day 120 and splenocytes or sub-populations were purified as previously described (16). Cell transfers were performed by i.v. injection of total splenocytes or purified sub-populations on the day of transplantation of LEW.1W onto LEW.1A sublethally irradiated (4.5 Gy day -1) recipient.

**Monoclonal antibodies and staining:** T cells and pDC were purified and sorted as previously described (20). Flow cytometry and cell sorting was performed using antibodies directed to T cells (TCRαβ, clone R7/3), CD4⁺CD25⁻ T cells (clones OX35 and OX39), CD8⁺ T cells (clone OX8), CD8⁺ or CD4⁺ CD45RC^{low} T cells (clones OX8 and OX22), and CD4⁺CD45R⁺85C7⁺ pDCs (clones His24, OX35 and 85C7), CDllb and CD45RA⁺ B cells (OX33) obtained from the European Collection of Cell Culture (Salisbury, UK). All biotin-labelled mAbs were visualized using Strepavidin-PE-Cy7 (BD Biosciences) or Streptavidin-Alexa405. A Canto II cytometer (BD Biosciences, Mountain View, CA) was used to measure fluorescence, and data were analyzed using the FLOWJO software (Tree Star, Inc. USA). Cells were first gated by their morphology excluding dead cells by selecting DAPI viable cells.

**Mixed lymphocyte reaction:** Naive Lewis 1A CD4⁺ T cells, naive Lewis 1W allogeneic pDC, and treated-syngeneic Lewis 1A CD8⁺CD45RC^{low}Tregs subsets were sorted as previously described (19). Proliferation of CFSE-labelled CD4⁺CD25⁻ T cells was analyzed by flow cytometry 6 days later, by gating on TCR⁺CD4⁺ cells (R7/3-APC, Ox35-PECY7) among living cells (DAPI negative).

**Antibody detection:** *Alloantibodies.* Donor spleens were digested by collagenase D, stopped with 400µl EDTA 0.1mM, and red cells were lysed. Serum of recipients were added to donor splenocytes at a dilution 1/8, and incubated with either anti-rat IgG-FITC (Jackson ImmunoResearch Labs INC, Baltimore, USA), anti-rat IgG1 (MCA 194, Serotec), anti-rat IgG2a (MCA 278, Serotec), or anti-rat IgG2b (STAR114F, Serotec) and anti-Ms Ig-FITC(115-095-164, Jackson ImmunoResearch). A FACS Canto (BD Biosciences, Mountain View, CA) was used to measure fluorescence, and data were analyzed using the FLOWJO software (Tree Star, Inc. USA). Geometric mean of fluorescence (MFI) of tested sera was divided by mean of 5 naive Lewis 1A sera MFI as control.

**Immunization and detection of anti-KLH antibodies:** Keyhole limpet hemocyanin (KLH, Sigma Aldrich, St. Louis, MO) was injected at >100 days after transplantation in the footpad (50 µg emulsified in 200 µl of complete freund adjuvant). Anti-KLH IgM and IgG Abs were detected repectively at day 4 and day 13 post-immunization by ELISA as previously described (18).

**Statistical analysis:** One Way ANOVA Kruskal Wallis test and Dunn's posttest was used for coculture experiments, Two-Way ANOVA test and Bonferroni posttests was applied for donor-directed antibodies , and splenocytes phenotype characterization, and Mantel Cox test was used to analyse survival curves.

### Results

**Tolerance induction following short-term anti-CD45RC antibody treatment:** As we previously demonstrated the presence and potential of CD8⁺ T cells with absent or low expression of the CD45RC surface marker, we speculated that a targeting strategy using an anti-CD45RC MAb would promote tolerance in cardiac transplanted recipients. Thus, we have administered an anti-CD45RC antibody (OX22, 0.8 mg/kg/i.v.) every 2.5 days during 20, 10 or 5 days to transplanted rat recipients (cardiac allograft MHC mismatched, LEW.1W grafted onto LEW.1A), starting the day of the transplantation. We obtained indefinite allograft survival in recipients treated 20 or 10 days with the anti-CD45RC antibody compared to control untreated recipients and recipients treated 5 days **(****Figure 1****)**, demonstrating for the first time that short-term therapy with the anti-CD545RC antibody efficiently promote indefinite allograft survival.

To further test the tolerogenic potential of the anti-CD45RC antibody in vivo, recipients were grafted in a stronger combination of allograft rejection (LEW.1A grafted onto LEW.1W) and administered the anti-CD45RC antibody for 20 days. In this graft combination, we also observed indefinite allograft survival in all recipients **(****Figure 1****),** demonstrating the high suppressive effect of the anti-CD45RC treatment.

Cardiac histopathology analysis revealed a complete absence of fibrosis, infiltrate and vessel sclerosis at day 120 in 20-days anti-CD45RC-treated recipients, demonstrating that this short-term treatment efficiently inhibits both acute and chronic rejection.

Finally, we analyzed in these recipients the presence of anti-donor antibodies and the capacity to mount antibody responses against exogenous antigens (KLH) at day 120 post-transplantation. We observed a complete absence of anti-donor IgG, IgG1, IgG2a and IgG2B antibodies in recipients treated 20 days with the anti-CD45RC antibody compared to controls. In contrast, we observed that immunization at day 120 of 20-days treated long-term surviving recipients with exogenous antigen (KLH mixed with CFA) resulted in high-level production of both IgM and IgG antibodies compared to naive recipients.

Altogether, we demonstrated that anti-CD45RC antibody treatment resulted in inhibition of both acute and chronic rejection, without compromising immunity and capacity of the recipients to mount antibody responses against cognate antigens.

**Short-term anti-CD45RC antibody treatment resulted in temporary lymphocyte reduction but increased regulatory populations:** To analyze the effect of the anti-CD45RC antibody on the phenotype of the cell subsets in the blood of 10-days treated-recipients at day 0, 4 and 10 after transplantation, we analyzed the different cell subset **(****Figure 2****).** We observed at day 4 a complete absence of CD45RC⁺ cells and a slight decreased of T and B cells (as CD45RC is expressed by both populations). At day 10, we observed all subset had return to normal level and we observed a strong increased in T CD4⁺CD45RC⁻, T CD8⁺CD45RC⁻ and B⁺CD45RC⁻ populations, as well as MDSCs **(****Figure 2****).**

Given the long-term tolerance induction that resulted from the 10 and 20-days anti-CD45RC-treatment, we analyzed the proportion of regulatory T⁺CD4⁺CD45RC^{-/low}, T⁺CD8⁺CD45RC^{-/low} or B⁺CD45RC^{-/low} cells in the spleen. At day 120 after treatment, analysis of the phenotype of the recipients in the spleen revealed a strong enrichment in absolute number of T cells and particularly of T CD4⁺CD45RC^{int/-}, T CD8⁺CD45RC⁻, while other cell subsets were not modified **(****Figure 3****).**

Altogether, we demonstrated that short-term anti-CD45RC treatment resulted in temporary decreased of CD45RC⁺, T and B cells, while T⁺CD4⁺CD45RC^{-/low}, T⁺CD8⁺CD45RC^{-/low} and B⁺CD45RC^{-/low} were increased early and at day 120 suggesting a role for regulatory cells in tolerance induction.

**Short-term anti-CD45RC antibody treatment resulted in potentiation of CD8⁺CD45RC^{-/low} Tregs and transferable tolerance:** To confirm *in vivo* that short-term treatment with anti-CD45RC antibodies resulted in induction of potent suppressive regulatory cells, we performed adoptive cell transfer of splenocytes or sub-populations into secondary irradiated cardiac grafted recipients **(****Figure 4****).** We observed that adoptive cell transfer of 150.10^{e6} splenocytes resulted in indefinite allograft survival in all recipients compared to recipients transferred with naive splenocytes or non-transferred. To further determine the subpopulation responsible of the tolerance induction, we sorted CD8⁺CD45RC^{-/low}, CD4⁺CD45RC^{- /low} and B⁺CD45RC^{-/low} cells and performed adoptive cell transfer. While we observed a slight prolongation of allograft survival in recipients adoptively transferred with B cells, we observed 100% allograft survival in recipients transferred with CD8⁺CD45RC^{low} Tregs and, CD4⁺CD45RC^{low} T cells, demonstrating that the depletion of CD45RC⁺ cells from day 0 to 20 has induced and activated regulatory cells with a strong suppressive capacity.

As we had previously described an *ex vivo* assay to characterize the suppressive activity of CD8⁺CD45RC^{low} Tregs (19) and to further evaluate and compare the suppressive capacity of CD8⁺CD45RC^{low} Tregs induced with the anti-CD45RC Ab to naive CD8⁺CD45RC^{low} Tregs, CD8⁺CD45RC^{low} Tregs were sorted at day 120 and added in a dose dependent manner in an assay were CFSE-labeled CD4⁺CD25⁻ effector T cells were cocultured with donor-derived plasmacytoid dendritic cells (pDC) **(****Figure 5****).** In absence of Tregs, we observed a strong proliferation of the CD4⁺CD25⁻ Teff cells. This proliferation was reduced in presence of naive CD8⁺CD45RC^{low} Tregs as we have previously described (19). Interestingly, in presence of anti-CD45RC-induced CD8⁺CD45RC^{low} Tregs from d120 recipients, we observed an almost total inhibition of proliferation of the CD4⁺CD25⁻ Teff cells in a dose dependent manner until at least an effector:suppressor ratio of 16:1, demonstrating the strong potential of the Ab therapy in inducing strong suppressive CD8⁺CD45RC^{low} Tregs.

### EXAMPLE 2: Anti-CD45RC mAb treatment reduces lethality and weight loss in a model of GVHD.

### Material & Methods

**Graft-versus-host disease induction *in vivo:*** 200.10⁶ splenocytes were injected intravenously in syngeneic or allogeneic rats treated 24 hours earlier with whole-body sublethal irradiation of 7.8 Gy. Recipients received anti-CD45RC mAbs (OX22) or an irrelevant control mAbs starting day 0 and every 3 days at 2mg/kg/injection. Recipients were weighted every day and sacrifice when percentage of weight loss was >20% of their initial weight.

### Results

**Anti-CD45RC mAb treatment reduces lethality and weight loss in a model of GVHD:** To determine whether administration of the anti-CD45RC could prevent the development of acute graft versus host disease (GVHD), recipients received either syngeneic or allogeneic splenocytes following whole-body sublethal irradiation. Anti-CD45RC or irrelevant control mAbs were then administered i.p. every 3 days. We observed that recipients that received syngeneic splenocytes started to lose weight but then recovered from the whole-body irradiation and gained weight until the end of the experiment. Interestingly, we observed that recipients that were administered allogeneic splenocytes and the anti-CD45RC antibody lost significatively less weight than the recipients administered the control irrelevant antibody and even started to gain weight by day 10, while the control group had lost >20% of their weight and was sacrificed by day 12, altogether demonstrating the potential of the anti-CD45RC therapy in controlling the GVHD **(****Figure 6****).**

### REFERENCES:

Throughout this application, various references describe the state of the art to which this present disclosure pertains.
   1. Gurkan, S., Luan, Y., Dhillon, N., Allam, S.R., Montague, T., Bromberg, J.S., Ames, S., Lerner, S., Ebcioglu, Z., Nair, V., et al. 2010. Immune reconstitution following rabbit antithymocyte globulin. Am J Transplant 10:2132-2141.
   2. Mai, H.L., Boeffard, F., Longis, J., Danger, R., Martinet, B., Haspot, F., Vanhove, B., Brouard, S., and Soulillou, J.P. 2014. IL-7 receptor blockade following T cell depletion promotes long-term allograft survival. J Clin Invest 124:1723-1733.
   3. Streuli, M., Hall, L.R., Saga, Y., Schlossman, S.F., and Saito, H. 1987. Differential usage of three exons generates at least five different mRNAs encoding human leukocyte common antigens. J Exp Med 166:1548-1566.
   4. Thomas, M.L. 1989. The leukocyte common antigen family. Annu Rev Immunol 7:339-369.
   5. Ordonez, L., Bernard, I., L'Faqihi-Olive, F.E., Tervaert, J.W., Damoiseaux, J., and Saoudi, A. 2009. CD45RC isoform expression identifies functionally distinct T cell subsets differentially distributed between healthy individuals and AAV patients. PLoS One 4:e5287.
   6. Birkeland, M.L., Johnson, P., Trowbridge, I.S., and Pure, E. 1989. Changes in CD45 isoform expression accompany antigen-induced murine T-cell activation. Proc Natl Acad Sci USA 86:6734-6738.
   7. Hermiston, M.L., Xu, Z., and Weiss, A. 2003. CD45: a critical regulator of signaling thresholds in immune cells. Annu Rev Immunol 21:107-137.
   8. Hargreaves, M., and Bell, E.B. 1997. Identical expression of CD45R isoforms by CD45RC+ 'revertant' memory and CD45RC+ naive CD4 T cells. Immunology 91:323-330.
   9. Lazarovits, A.I., Poppema, S., Zhang, Z., Khandaker, M., Le Feuvre, C.E., Singhal, S.K., Garcia, B.M., Ogasa, N., Jevnikar, A.M., White, M.H., et al. 1996. Prevention and reversal of renal allograft rejection by antibody against CD45RB. Nature 380:717-720.
   10. Basadonna, G.P., Auersvald, L., Khuong, C.Q., Zheng, X.X., Kashio, N., Zekzer, D., Minozzo, M., Qian, H., Visser, L., Diepstra, A., et al. 1998. Antibody-mediated targeting of CD45 isoforms: a novel immunotherapeutic strategy. Proc Natl Acad Sci U S A 95:3821-3826.
   11. Abu-Hadid, M.M., Lazarovits, A.I., and Madrenas, J. 2000. Prevention of diabetes mellitus in the non-obese diabetic mouse strain with monoclonal antibodies against the CD45RB molecule. Autoimmunity 32:73-76.
   12. Rogers, P.R., Pilapil, S., Hayakawa, K., Romain, P.L., and Parker, D.C. 1992. CD45 alternative exon expression in murine and human CD4+ T cell subsets. J Immunol 148:4054-4065.
   13. Spickett, G.P., Brandon, M.R., Mason, D.W., Williams, A.F., and Woollett, G.R. 1983. MRC OX-22, a monoclonal antibody that labels a new subset of T lymphocytes and reacts with the high molecular weight form of the leukocyte-common antigen. J Exp Med 158:795-810.
   14. Xystrakis, E., Bernard, I., Dejean, A.S., Alsaati, T., Druet, P., and Saoudi, A. 2004. Alloreactive CD4 T lymphocytes responsible for acute and chronic graft-versus-host disease are contained within the CD45RChigh but not the CD45RClow subset. Eur J Immunol 34:408-417.
   15. Xystrakis, E., Dejean, A.S., Bernard, I., Druet, P., Liblau, R., Gonzalez-Dunia, D., and Saoudi, A. 2004. Identification of a novel natural regulatory CD8 T-cell subset and analysis of its mechanism of regulation. Blood 104:3294-3301.
   16. Guillonneau, C., Hill, M., Hubert, F.X., Chiffoleau, E., Herve, C., Li, X.L., Heslan, M., Usal, C., Tesson, L., Menoret, S., et al. 2007. CD40Ig treatment results in allograft acceptance mediated by CD8CD45RC T cells, IFN-gamma, and indoleamine 2,3-dioxygenase. J Clin Invest 117:1096-1106.
   17. Powrie, F., and Mason, D. 1990. OX-22high CD4+ T cells induce wasting disease with multiple organ pathology: prevention by the OX-22low subset. J Exp Med 172:1701-1708.
   18. Guillot, C., Guillonneau, C., Mathieu, P., Gerdes, C.A., Menoret, S., Braudeau, C., Tesson, L., Renaudin, K., Castro, M.G., Lowenstein, P.R., et al. 2002. Prolonged blockade of CD40-CD40 ligand interactions by gene transfer of CD40Ig results in long-term heart allograft survival and donor-specific hyporesponsiveness, but does not prevent chronic rejection. J Immunol 168:1600-1609.
   19. Li, X.L., Menoret, S., Bezie, S., Caron, L., Chabannes, D., Hill, M., Halary, F., Angin, M., Heslan, M., Usal, C., et al. 2010. Mechanism and localization of CD8 regulatory T cells in a heart transplant model of tolerance. J Immunol 185:823-833.
   20. Picarda, E., Bezie, S., Venturi, V., Echasserieau, K., Merieau, E., Delhumeau, A., Renaudin, K., Brouard, S., Bernardeau, K., Anegon, I., et al. 2014. MHC-derived allopeptide activates TCR-biased CD8+ Tregs and suppresses organ rejection. J Clin Invest 124:2497-2512.
   21. Ordonez, L., Bernard, I., Chabod, M., Augusto, J.F., Lauwers-Cances, V., Cristini, C., Cuturi, M.C., Subra, J.F., and Saoudi, A. 2013. A higher risk of acute rejection of human kidney allografts can be predicted from the level of CD45RC expressed by the recipients' CD8 T cells. PLoS One 8:e69791.
   22. Haspot, F., Seveno, C., Dugast, A.S., Coulon, F., Renaudin, K., Usal, C., Hill, M., Anegon, I., Heslan, M., Josien, R., et al. 2005. Anti-CD28 antibody-induced kidney allograft tolerance related to tryptophan degradation and TCR class II B7 regulatory cells. Am J Transplant 5:2339-2348.
   23. Josien, R., Heslan, M., Brouard, S., Soulillou, J.P., and Cuturi, M.C. 1998. Critical requirement for graft passenger leukocytes in allograft tolerance induced by donor blood transfusion. Blood 92:4539-4544.
   24. Guillonneau, C., Louvet, C., Renaudin, K., Heslan, J.M., Heslan, M., Tesson, L., Vignes, C., Guillot, C., Choi, Y., Turka, L.A., et al. 2004. The role of TNF-related activation-induced cytokine-receptor activating NF-kappa B interaction in acute allograft rejection and CD40L-independent chronic allograft rejection. J Immunol 172:1619-1629.
   25. Bedke T, Baars W, Schwinzer R. Modulation of human anti-pig T cell responses by monoclonal antibodies directed to porcine CD45 molecules. Ann Transplant. 2003;8(3):35-8.

## Claims

1. An isolated anti-CD45RC monoclonal antibody for use in preventing or reducing transplant rejection in a patient in need thereof, wherein the anti-CD45RC monoclonal antibody is a CD45RC+ cell depleting antibody.

2. The anti-CD45RC monoclonal antibody for use according to claim 1 wherein said transplant rejection is selected from the group consisting of allogeneic hematopoietic stem cells rejection (GVHD), cardiac, pancreatic islet, vascular tissue, kidney, lung and liver transplant rejection in a patient in need thereof.

3. The anti-CD45RC monoclonal antibody for use according to claim 1, wherein said transplant rejection is GVHD.

4. The anti-CD45RC monoclonal antibody for use according to claim 2, wherein said transplant rejection is cardiac allotransplant rejection.

5. The anti-CD45RC monoclonal antibody for use according to claim 1, wherein said anti-CD45RC monoclonal antibody is an anti-human CD45RC monoclonal antibody.

6. The anti-CD45RC monoclonal antibody for use according to claim 1, wherein said anti-CD45RC monoclonal antibody is a humanized antibody or a fully human antibody.

7. The anti-CD45RC monoclonal antibody for use according to claim 1, wherein said anti-CD45RC monoclonal antibody is a chimeric antibody.

8. The anti-CD45RC monoclonal antibody for use according to claim 1 wherein said anti-CD45RC monoclonal antibody mediates antibody-dependent cell mediated cytotoxicity (ADCC) and/or complement dependent cytotoxicity (CDC).

9. The anti-CD45RC monoclonal antibody for use according to claim 1 wherein said anti-CD45RC monoclonal antibody is an IgG1 isotype.

10. The anti-CD45RC monoclonal antibody for use according to claim 1 wherein said anti-CD45RC monoclonal antibody is conjugated to a cytotoxic agent or a growth inhibitory agent.

11. The anti-CD45RC monoclonal antibody for use according to claim 1 wherein the transplantation is syngeneic, allogeneic or xenogeneic.

12. The anti-CD45RC monoclonal antibody for use according to claim 1 wherein said transplant rejection is an acute or a chronic transplant rejection.

13. The anti-CD45RC monoclonal antibody for use according to claim 1 wherein the patient is transplanted with allogeneic hematopoietic stem cells for the treatment of a disease selected from the group consisting of acute myeloid leukemia (AML); acute lymphoid leukemia (ALL); chronic myeloid leukemia (CML); myelodysplasia syndrome (MDS) / myeloproliferative syndrome; lymphomas such as Hodgkin and non-Hodgkin lymphomas, chronic lymphatic leukemia (CLL) and multiple myeloma.

14. A pharmaceutical composition or a kit-of-part for use in preventing or treating allogeneic transplant rejection comprising an isolated anti-CD45RC monoclonal antibody that is a CD45RC+ cell depleting antibody and an immunosuppressive drug selected from the group consisting of cytostatics, alkylating agents, antimetabolites therapeutic antibodies; calcineurin inhibitors; glucocorticoids and mycophenolate mofetil.

## Patentansprüche

1. Isolierter monoklonaler Anti-CD45RC-Antikörper zur Verwendung beim Verhindern oder Reduzieren der Transplantatabstoßung bei einem Patienten, der dieser bedarf, wobei der monoklonale Anti-CD45RC-Antikörper ein CD45RC+ Zellen abbauender Antikörper ist.

2. Monoklonaler Anti-CD45RC-Antikörper zur Verwendung nach Anspruch 1, wobei die Transplantatabstoßung aus der Gruppe ausgewählt ist, bestehend aus allogener hämatopoetischer Stammzellenabstoßung (GVHD), Herz-, Pankreasinsel-, Gefäßgewebe-, Nieren-, Lungen- und Lebertransplantationabstoßung, bei einem Patienten, der dessen bedarf.

3. Monoklonaler Anti-CD45RC-Antikörper zur Verwendung nach Anspruch 1, wobei die Transplantatabstoßung GVHD ist.

4. Monoklonaler Anti-CD45RC-Antikörper zur Verwendung nach Anspruch 2, wobei die Transplantatabstoßung kardiale Allotransplantatabstoßung ist.

5. Monoklonaler Anti-CD45RC-Antikörper zur Verwendung nach Anspruch 1, wobei der monoklonale Anti-CD45RC-Antikörper ein monoklonaler Anti-Human-CD45RC-Antikörper ist.

6. Monoklonaler Anti-CD45RC-Antikörper zur Verwendung nach Anspruch 1, wobei der monoklonale Anti-CD45RC-Antikörper ein humanisierter Antikörper oder ein vollständig humaner Antikörper ist.

7. Monoklonaler Anti-CD45RC-Antikörper zur Verwendung nach Anspruch 1, wobei der monoklonale Anti-CD45RC-Antikörper ein chimärer Antikörper ist.

8. Monoklonaler Anti-CD45RC-Antikörper zur Verwendung nach Anspruch 1, wobei der monoklonale Anti-CD45RC-Antikörper antikörper-abhängige zellvermittelte Cytotoxizität (ADCC) und/oder komplement-abhängige Cytotoxizität (CDC) vermittelt.

9. Monoklonaler Anti-CD45RC-Antikörper zur Verwendung nach Anspruch 1, wobei der monoklonale Anti-CD45RC-Antikörper ein lgG-1-lsotyp ist.

10. Monoklonaler Anti-CD45RC-Antikörper zur Verwendung nach Anspruch 1, wobei der monoklonale Anti-CD45RC-Antikörper an ein cytotoxisches Mittel oder ein wachstumshemmendes Mittel konjugiert ist.

11. Monoklonaler Anti-CD45RC-Antikörper zur Verwendung nach Anspruch 1, wobei die Transplantation syngen, allogen oder xenogen ist.

12. Monoklonaler Anti-CD45RC-Antikörper zur Verwendung nach Anspruch 1, wobei die Transplantatabstoßung eine akute oder eine chronische Transplantatabstoßung ist.

13. Monoklonaler Anti-CD45RC-Antikörper zur Verwendung nach Anspruch 1, wobei dem Patienten allogene hämatopoetische Stammzellen zur Behandlung einer Krankheit, die ausgewählt ist aus der Gruppe, bestehend aus akuter myeloischer Leukämie (AML); akuter lymphoider Leukämie (ALL); chronischer myeloischer Leukämie (CML); Myelodysplasie-Syndrom (MDS)/myeloproliferativem Syndrom; Lymphomen wie Hodgkin- und Nicht-Hodgkin-Lymphomen, chronischer lymphatischer Leukämie (CLL) und multiplem Myelom, transplantiert werden.

14. Pharmazeutische Zusammensetzung oder Kit-of-Part zur Verwendung bei der Verhinderung oder Behandlung allogener Transplantatabstoßung, die einen isolierten monoklonalen Anti-CD45RC-Antikörper, der ein CD45RC+ Zellen abbauender Antikörper ist, und ein Immunsuppressivum, ausgewählt aus der Gruppe, bestehend aus Cytostatika, Alkylierungsmitteln, Antimetaboliten, therapeutischen Antikörpern; Calcineurininhibitoren; Glucocorticoiden und Mycophenolat-Mofetil, umfasst.

## Revendications

1. Anticorps monoclonal isolé anti-CD45RC destiné à être utilisé dans la prévention ou la réduction d'un rejet de greffe chez un patient en ayant besoin, dans lequel l'anticorps monoclonal anti-CD45RC est un anticorps dépletant les cellules CD45RC+.

2. Anticorps monoclonal anti-CD45RC destiné à être utilisé selon la revendication 1, dans lequel ledit rejet de greffe est sélectionné à partir du groupe constitué par le rejet de cellules souches hématopoïétiques allogéniques (GVH), un rejet de greffe cardiaque, d'îlots pancréatiques, de tissu vasculaire, de rein, de poumon et de foie chez un patient en ayant besoin.

3. Anticorps monoclonal anti-CD45RC destiné à être utilisé selon la revendication 1, dans lequel ledit rejet de greffe est la GVH.

4. Anticorps monoclonal anti-CD45RC destiné à être utilisé selon la revendication 2, dans lequel ledit rejet de greffe est un rejet d'allogreffe cardiaque.

5. Anticorps monoclonal anti-CD45RC destiné à être utilisé selon la revendication 1, dans lequel ledit anticorps monoclonal anti-CD45RC est un anticorps monoclonal anti-CD45RC humain.

6. Anticorps monoclonal anti-CD45RC destiné à être utilisé selon la revendication 1, dans lequel ledit anticorps monoclonal anti-CD45RC est un anticorps humanisé ou un anticorps entièrement humain.

7. Anticorps monoclonal anti-CD45RC destiné à être utilisé selon la revendication 1, dans lequel ledit anticorps monoclonal anti-CD45RC est un anticorps chimérique.

8. Anticorps monoclonal anti-CD45RC destiné à être utilisé selon la revendication 1, dans lequel ledit anticorps monoclonal anti-CD45RC induit une cytotoxicité à médiation cellulaire dépendante de l'anticorps (ADCC) et/ou une cytotoxicité dépendante du complément (CDC).

9. Anticorps monoclonal anti-CD45RC destiné à être utilisé selon la revendication 1, dans lequel ledit anticorps monoclonal anti-CD45RC est un isotype lgG1.

10. Anticorps monoclonal anti-CD45RC destiné à être utilisé selon la revendication 1, dans lequel ledit anticorps monoclonal anti-CD45RC est conjugué à un agent cytotoxique ou à un agent d'inhibition de croissance.

11. Anticorps monoclonal anti-CD45RC destiné à être utilisé selon la revendication 1, dans lequel la greffe est syngénique, allogénique ou xénogénique.

12. Anticorps monoclonal anti-CD45RC destiné à être utilisé selon la revendication 1, dans lequel ledit rejet de greffe est un rejet de greffe aigu ou chronique.

13. Anticorps monoclonal anti-CD45RC destiné à être utilisé selon la revendication 1, dans lequel le patient est greffé par des cellules souches hématopoïétiques allogéniques pour le traitment d'une maladie sélectionnée à partir du groupe constitué par la leucémie myéloïde aiguë (LMA) ; la leucémie lymphoïde aiguë (LLA) ; par la leucémie myéloïde chronique (LMC) ; le syndrome myélodisplasique (SMD) / le syndrome myéloprolifératif ; les lymphomes, tels que les lymphomes de Hodgkin et non-hodgkinien, la leucémie lymphatique chronique (LLC) et le myélome multiple.

14. Composition pharmaceutique ou un kit d'éléments destiné à être utilisé dans la prevention ou le traitement d'un rejet de greffe allogénique comprenant un anticorps monoclonal isolé anti-CD45RC qui est un anticorps de déplétion de cellules CD45RC+ et un médicament immunosuppresseur sélectionné à partir du groupe constitué par les cytostatiques, les agents alkylants, les antimétabolites, les anticorps thérapeutiques, les inhibiteurs de la calcineurine, les glycocorticoïdes et le mycophénolate mofétil.
